# EUROPÄISCHE PATENTSCHRIFT

(11) **EP 1 633 258 B1**
(45) Veröffentlichungstag und Bekanntmachung des Hinweises auf die Patenterteilung: **20.12.2006**
(21) Anmeldenummer: 04713471.3
(22) Anmeldetag: 21.02.2004
(51) Int. Cl.: A61B 17/68, F16B 13/02

(54) **Knochendübel**
Bone plug
CHEVILLE OSSEUSE

(30) Priorität: 09.04.2003 DE 20305713 U
(43) Veröffentlichungstag der Anmeldung: 15.03.2006
(73) Patentinhaber: GAUSEPOHL, Thomas, 51109 Köln (DE); Willmen, Hans Rainer, Prof. Dr. med., 41515 Grevenbroich (DE)
(72) Erfinder: GAUSEPOHL, Thomas, 51109 Köln (DE); Willmen, Hans Rainer, Prof. Dr. med., 41515 Grevenbroich (DE)
(74) Vertreter: Koch, Günther
(86) Internationale Anmeldenummer: PCT/EP2004/001728
(87) Internationale Veröffentlichungsnummer: WO 2004/089230

(56) Entgegenhaltungen:
- EP-A- 0 142 474
- EP-A- 0 340 159
- EP-A- 0 528 573
- FR-A- 2 691 626

## Beschreibung

Die Erfindung bezieht sich auf einen Knochendübel, der in eine Bohrung im Knochen einsteckbar ist und in den eine beispielsweise aus Edelstahl oder resorbierbarem Material bestehende Schraube eingedreht werden kann, um eine zuverlässige, dauerhafte Verankerung im Knochen zu gewährleisten. Derartige Knochendübel werden bei verschiedenen Operationstechniken benutzt. Ein Anwendungsfall ist der Einsatz derartiger Dübel für die Fixation von Knochenfragmenten mittels einer Osteosynthese-Platte, wie dies in der DE 101 07 201 beschrieben ist. Derartige Knochendübel können auch eingesetzt werden zur Verankerung von Sehnen in einem Knochenkanal, um beispielsweise Gelenke nach erfolgtem Kapsel-/Bandriss zu stabilisieren, wie dies im Einzelnen in der DE 100 35 610 A1 beschrieben ist.

Knochendübel finden weiter Verwendung zur Fixierung eines Fadens, mit dessen Hilfe Gewebe an einem Knochen befestigt werden kann. Ein derartiger Dübel ist in der EP 0 502 509 A1 beschrieben.

Die DE 35 09 417 A1 beschreibt eine Einrichtung zur Unterstützung der Osteosynthese in der Knochenchirurgie, bei welcher ein Knochendübel zum Eindrehen einer Schraube vorgesehen ist, bei dem ein kreiszylindrischer Dübelmantel ein Durchgangsloch mit über die Länge gleichem Querschnitt aufweist und der Dübelmantel längs einer Mantellinie durch einen Längsschlitz unterbrochen ist. Dieser Längsschlitz teilt den unteren Abschnitt des Dübels in der Weise, dass zwei getrennte Dübelbeine gebildet werden.

Die EP 0 340 159 A1 beschreibt einen Spreizdübel für eine zementfreie Verankerung von Knochenimplantaten, bei der ein Hohlkörper eines Spreizdübels zur Verankerung von Implantaten in Knochen entlang seines äußeren Umfangs polygonförmig ausgebildet ist.

Die DE 34 17 923 A1 beschreibt eine Endoprothese und einen Dübel für die Verankerung in einem Material mit knochenartiger Festigkeit. Die Endoprothese besteht aus einem hohlen aufspreizbaren Verankerungsteil, in dessen Hohlraum ein Spreizkörper einführbar ist, durch den der in eine Knochenbohrung eingesetzte Verankerungsteil an den Wänden der Knochenbohrung festgeklemmt und dadurch zementlos im Knochen verankert wird.

Der Erfindung liegt die Aufgabe zugrunde, einen Knochendübel zu schaffen, der für zahlreiche Anwendungsfälle der Operationstechnik einsetzbar ist und einen zuverlässigen Halt gewährleistet. Hierin besteht ein zunehmend großer Bedarf bei osteoporetischen Knochen aufgrund hormoneller Veränderungen im weiblichen Skelett und altersbedingtem Knochenabbau älter werdender Menschen. Gerade bei Osteoporose kommt es im Rahmen von Osteosynthesen zum Überdrehen von Schrauben, so dass z.B. Metallplatten nicht sicher verankert werden können, was ein Umsteigen auf andere Operationstechniken erforderlich macht.

Gelöst wird die gestellte Aufgabe durch die Gesamtheit der im Patentanspruch 1 angegebenen Merkmale.

Weitere zweckmäßige Ausgestaltungen ergeben sich aus den Unteransprüchen 2 bis 10.

Der Dübel kann in an sich bekannter Weise aus resorbierbarem Material bestehen, wobei die Schraube an der Knochenbohrungswandung festgelegt im Körper verbleiben oder operativ entfernt werden kann.

Der erfindungsgemäße Knochendübel erfüllt alle auf dem einschlägigen Spezialgebiet zu stellenden Anforderungen und kann in der allgemeinen Dübeltechnik kein Vorbild finden.

Nachstehend werden Ausführungsbeispiele der Erfindung anhand der Zeichnung beschrieben.

In der Zeichnung zeigen:
Fig. 1 ist eine perspektivische Darstellung eines erfindungsgemäßen Knochendübels;
Fig. 2 ist eine Ansicht des Knochendübels, betrachtet in Richtung der Schlitzebene;
Fig. 3 ist eine Stirnansicht des Dübels in Richtung des Pfeiles III gemäß Fig. 2;
Fig. 4 ist eine Stirnansicht des Dübels in Richtung des Pfeiles IV gemäß Fig. 2;
Fig. 5 ist ein Schnitt nach der Linie V-V gemäß Fig. 2 in größerem Maßstab gezeichnet;
Fig. 6 ist eine Ansicht des Dübels gemäß Fig. 2, um 90° verdreht;
Fig. 7 ist ein Axialschnitt des Knochendübels gemäß Fig. 1 bis 6;
Fig. 8 ist eine perspektivische Ansicht eines bevorzugten Ausführungsbeispiels eines Knochendübels gemäß der Erfindung.
Fig. 9 ist eine Schnittansicht von im Knochen verankerten, eine Osteosynthese-Platte halternden Dübeln.

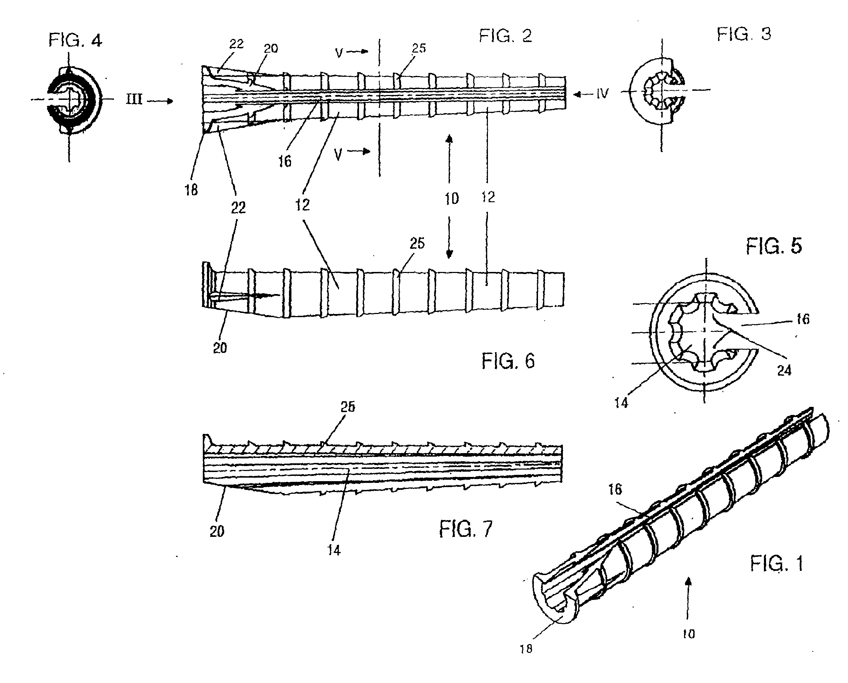

Der Knochendübel 10 gemäß Fig. 1 bis 7 besteht aus einem im Querschnitt kreisförmigen, außen schwach konisch verlaufenden Dübelmantel 12, der ein zylindrisches Durchgangsloch 14 umschließt und einen über eine Mantellinie verlaufenden durchgehenden Längsschlitz 16 aufweist. Der Außendurchmesser des Dübelmantels 12 nimmt vom Kopfteil nach dem Dübelfuß ab, wodurch das Einführen in die Knochenbohrung erleichtert wird. Bei einem Außendurchmesser von 5 mm im Kopfteil weist der Längsschlitz 16 eine Breite von etwa 1,5 mm auf. Das vom Dübelmantel 12 umschlossene Durchgangsloch 14 weist einen Durchmesser von etwa 4 mm auf. Am Kopfende ist der Dübelmantel 12 zu einem senkkopfartigen Kopfflansch 18 ausgeformt. Am Kopfende weist der Dübelmantel 12 eine Abschrägung 20 auf, die in der Mitte vom Schlitz 16 durchsetzt ist. Diese Abschrägung verläuft vom Kopfflansch nach den Außenumfang des Dübelmantels, so dass die Breite der Abschrägung, wie aus Fig. 2 ersichtlich, vom Kopfflansch 18 abnimmt. Auf der äußeren Oberfläche weist der Dübelmantel im Kopfteil als Verdrehsicherung drei im Winkelabstand von 120° zueinander angeordnete Längsrippen 22 (Finnen) auf, deren Höhe vom radial äußeren Durchmesser des Kopfflansches 18 nach dem Dübelmantel hin abnimmt und in den Mantel übergeht. Der Querschnitt der Längsrippen hat die Form eines spitzwinklig gleichschenkligen Dreicks.

Auf der Innenseite trägt der Dübelmantel 12 über die Länge durchgehende Stege 24 mit dem Querschnitt eines Kreisabschnitts.

Wie aus der Zeichnung ersichtlich, trägt der Dübelmantel 12 auf seiner Außenseite im axialen Abstand zueinander angeordnete Ringrippen 25, die in Umfangsrichtung verlaufen und scharfkantig ausgebildet sind. Insbesondere sind diese Ringrippen 25 widerhakenartig ausgebildet mit einer steilen nach dem Dübelkopf weisenden Vorlaufflanke 26 und einer flachen nach dem Dübelmantel hin verlaufenden Nachlaufflanke 28. Hierdurch wird mit Sicherheit ein axiales Verschieben innerhalb der Knochenbohrung beim Eindrehen der Schraube und danach verhindert, wobei jedoch über die flachen Flanken ein leichtes Einschieben möglich ist, während die steile Flanke der Rippen ein Ausziehen verhindert.

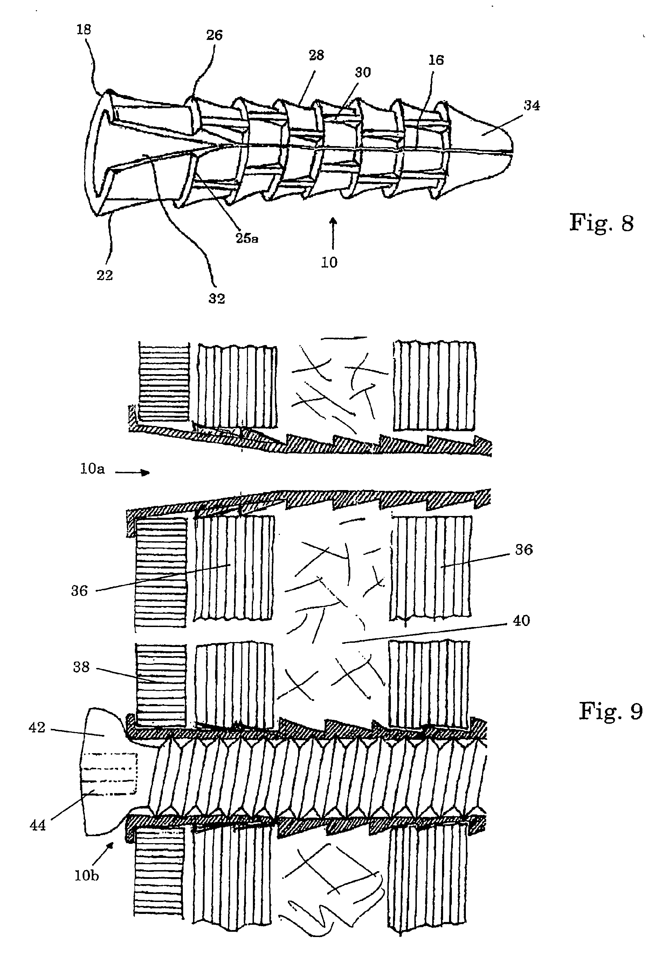

Das Ausführungsbeispiel gemäß Fig. 8 entspricht dem Ausführungsbeispiel nach Fig. 1 bis 7 mit dem Unterschied, dass die Ringrippen 25a konisch derart ausgebildet sind, dass ihre flachen Nachlaufflanken 28 jeweils bis zu den steilen Vorlaufflanken 26 der Ringrippen abfallen. Die Nachlaufflanken 28 tragen Längsstege 30, deren Höhe der maximalen Höhe der Ringrippen 25a entspricht und die jeweils gegenüber den Längsstegen der in axialer Richtung folgenden Nachlaufflanken 28 in Umfangsrichtung versetzt sind. Die Abschrägung 32 ist bei diesem Ausführungsbeispiel als V-förmige Einlauföffnung ausgebildet, die symmetrisch im Längsschlitz 16 mündet. Der Dübel weist einen balligen Dübelfuß 34 auf.

Der Kopfflansch 18 dient zur Auflage auf der Plattenoberfläche und es wird hierdurch verhindert, dass der Dübel in das Plattenloch rutscht. Die Längsrippen 22 in Gestalt von Finnen dienen der Verklemmung im Loch. Die abgeschrägte V-förmige Einlauföffnung 32 ermöglicht einen direkten Kontakt eines Schraubengewindeanteils und damit wird die Tendenz des Dübels, im Plattenschraubenloch zu rotieren, verhindert. Die Ringrippen 25a können sich nach dem Eindrehen der Schraube hinter die diesseitige und jenseitige Corticalis einhaken und so den Schraubenhals verbessern. Die Längsstege 30 werden beim Eindrehen der Schraube gegen die Corticalis gepresst und vermindern zusammen mit den Längsrippen 22 und der abgeschrägten Einlauföffnung 32 eine Rotation des Dübels im Knochen. Die Längsschlitze 16 erlauben eine Aufspreizung des Dübels. Die konisch-ballige Dübelspitze 34 erleichtert das Eindrehen des Dübels.

Fig. 9 zeigt die Verankerung eines Knochendübels gemäß Fig. 8 in der Knochencorticalis 36 zur Festlegung einer metallischen Osteosynthese-Platte 38. Wie aus Fig. 9 ersichtlich, durchstößt der Dübel die Knochenspongiose 40. In der Zeichnung ist der eingesteckte Knochendübel 10a vor dem Eindrehen der Schraube dargestellt und der Knochendübel 10b ist im aufgespreizten Zustand dargestellt, wobei die Aufspreizung durch eine Schraube 42 erfolgt ist, die über einen Innensechskant 44 angezogen wurde.

Wie ersichtlich, können die widerhakenartig ausgebildeten Ringrippen beim Einstecken des Knochendübels in das vorgebohrte Loch radial zurückweichen und nach Spreizung ergeben diese Ringrippen einen zuverlässigen Halt gegen Herausrutschen.

Die in der Zeichnung dargestellten Knochendübel können aus einem für chirurgische Zwecke geeigneten Kunststoff oder einem resorbierbaren Material bestehen. Es ist auch möglich, den Dübel und/oder die Schraube aus rostfreiem Stahl oder einem anderen geeigneten Metall, z.B. aus Titat, herzustellen. Der Dübel kann sich beim Einstecken in die Knochenbohrung infolge des Längsschlitzes im Durchmesser verringern, was das Einschieben in Verbindung mit den flachen Flanken der Umfangsrippen erleichtert. Ein Herausziehen mit oder ohne eingedrehter Schraube wird durch die steile Flanke des Widerhakens erschwert.

Der Dübel kann nach Abschluss der Knochenheilung zusammen mit der Schraube entfernt werden, sofern diese aus nicht-resorbierbarem Material bestehen. Wenn der Knochendübel und/oder die Schraube aus resorbierbarem Material bestehen, so wurden diese bereits während der Knochenheilung resorbiert und durch körpereigenes Knochengewebe ersetzt und verbleiben somit im Körper. Aus Metall bestehende Dübel und/oder Schrauben können entfernt oder unter medizinischen Aspekten auch im Körper belassen werden.

### Bezugszeichenliste

- 10: Knochendübel
- 12: Dübelmantel
- 14: Durchgangsloch
- 16: Längsschlitz
- 18: Kopfflansch
- 20: Abschrägung
- 22: Längsrippen
- 24: Stege
- 25: Ringrippen
- 25a: Ringrippen
- 26: Vorlaufflanke
- 28: Nachlaufflanke
- 30: Längsstege
- 32: Abschrägung V-förmige Einlauföffnung
- 34: balliger Dübelfuß
- 36: Knochencorticalis
- 38: Osteosynthese-Platte
- 40: Knochenspongiose
- 42: Schraube
- 44: Innensechskant

## Patentansprüche

1. Knochendübel (10) zum Eindrehen einer Schraube, beispielsweise zur gegenseitigen Festlegung von Knochenfragmenten, mittels einer Osteosynthese-Platte mit den folgenden Merkmalen:
- ein im Querschnitt kreisförmiger, außen schwach konischer, sich vom Dübelkopf nach dem Dübelfuß verjüngender Dübelmantel (12) weist ein Durchgangsloch (14) mit über die Länge gleichem Querschnitt auf;
- der Dübelmantel (12) ist längs einer Mantellinie durch einen Längsschlitz (16) unterbrochen, wodurch der Dübelmantel einen durchgehend C-förmigen Querschnitt erhält;
- am Kopfteil des Dübels ist ein senkkopfartiger Begrenzungskopfflansch (18) angeformt;
- der Kopfteil des Dübelmantels ist im Bereich des Längsschlitzes (16) mit einer Abschrägung versehen, deren Breite vom Kopfteil nach dem Dübelmantel abnimmt;
- der Dübelmantel weist im axialen Abstand über seine Gesamtlänge verteilt Ringrippen (25) auf.

2. Knochendübel (10) nach Anspruch 1,
**dadurch gekennzeichnet, dass** die Ringrippen (25) spitzwinklig und widerhakenartig ausgebildet sind und eine nach dem Kopfteil gerichtete steile Flanke (26) und eine nach dem Fußteil gerichtete flache Flanke (28) aufweisen.

3. Knochendübel (10) nach den Ansprüchen 1 und 2, bei welchem der Dübelmantel außerdem Längsrippen (22) und Längsstege (30) als Drehsicherung aufweist.

4. Knochendübel (10) nach den Ansprüchen 1 bis 3, bei welchem die Längsstege (30) jeweils zwischen den Ringrippen (25a) verlaufen, wobei ihre Außenkante in der maximalen radialen Höhe der Ringrippen verläuft.

5. Knochendübel (10) nach Anspruch 4, bei welchem die axial benachbarten Längsstege (30) jeweils in Umfangsrichtung gegeneinander versetzt sind.

6. Knochendübel (10) nach Anspruch 2, bei welchem die nach dem Fußteil gerichteten flachen Nachlaufflanken (28) konusförmig ausgebildet jeweils bis zur steilen Vorlaufflanke (26) der folgenden Ringrippe (25a) verlaufen.

7. Knochendübel (10) nach Anspruch 1, bei welchem die Abschrägung als V-förmige Einlauföffnung (32) des Längsschlitzes (16) ausgebildet ist.

8. Knochendübel (10) nach den Ansprüchen 1 und 3, bei dem der Dübelmantel im Kopfteil Längsrippen (22) aufweist, deren Höhe vom Kopfflansch (18) nach der ersten Querrippe abnimmt.

9. Knochendübel (10) nach Anspruch 1, bei welchem die letzte Ringrippe im Fußteil zu einem ballig geformten Dübelfuß (34) übergeht.

10. Knochendübel (10) nach den Ansprüchen 1 bis 9, bei welchem der Dübel und/oder die Schraube aus resorbierbarem Material besteht.

## Claims

1. Bone dowel (10) for the screwing in of a screw, for example for the reciprocal fixing of bone fragments, by means of an osteosynthesis plate with the following features:
- a dowel casing (12), circular in cross-section, outwardly slightly conical, tapering from the dowel head to the dowel base, has a continuous hole (14) having the same cross-section over its length;
- the dowel casing (12) is broken along a casing line by a longitudinal slit (16), whereby the dowel casing receives a continuously C-shaped cross-section;
- a countersunk-type limiting head flange (18) is moulded on the head of the dowel;
- the head of the dowel casing is provided in the area of the longitudinal slit (16) with a chamfer the width of which decreases from the head to the dowel casing;
- the dowel casing has circular ribs (25) distributed axially spaced over its entire length.

2. Bone dowel (10) according to claim 1,
**characterized in that** the circular ribs (25) are sharp-edged and barb-like and have a steep flank (26) pointing towards the head and a flat flank (28) pointing towards the foot.

3. Bone dowel (10) according to claims 1 and 2, in which the dowel casing additionally has longitudinal ribs (22) and longitudinal fins (30) as rotation-resistance means.

4. Bone dowel (10) according to claims 1 to 3, in which the longitudinal fins (30) each run between the circular ribs (25a), wherein their outer edge runs at the maximum radial height of the circular ribs.

5. Bone dowel (10) according to claim 4, in which the axially adjacent longitudinal fins (30) are each offset vis-à-vis each other in circumferential direction.

6. Bone dowel (10) according to claim 2, in which each of the flat trailing flanks (28) pointing towards the foot runs, formed conical, as far as the steep leading flank (26) of the following circular rib (25a).

7. Bone dowel (10) according to claim 1, in which the chamfer is formed as a V-shaped inlet opening (32) of the longitudinal slit (16).

8. Bone dowel (10) according to claims 1 and 3, in which in the head, the dowel casing has longitudinal ribs (22) the height of which decreases from the head flange (18) to the first cross-rib.

9. Bone dowel (10) according to claim 1, in which the last circular rib in the foot passes into a domed dowel base (34).

10. Bone dowel (10) according to claims 1 to 9, in which the dowel and/or the screw consists of resorbable material.

## Revendications

1. Cheville osseuse (10) pour y visser une vis, par exemple afin de fixer des fragments osseux l'un à l'autre au moyen d'une plaque pour ostéosynthèse, comprenant les caractéristiques suivantes :
- une enveloppe de cheville (12) de section circulaire et légèrement conique à l'extérieur, qui se rétrécit du haut de la cheville vers le bas de celle-ci, présente un trou (14) la traversant de part en part dont la section transversale est constante sur toute la longueur ;
- l'enveloppe de cheville (12) est interrompue le long d'une génératrice par une fente longitudinale (16) qui confère à l'enveloppe de cheville une section transversale de bout en bout en forme de C ;
- à l'extrémité supérieure de la cheville est ménagée une collerette de délimitation (18) semblable à une tête conique ;
- la partie supérieure de l'enveloppe de cheville est dotée, dans la zone de la fente longitudinale (16), d'un chanfrein dont la largeur va en diminuant de la partie supérieure vers l'enveloppe de cheville ;
- l'enveloppe de cheville présente des nervures annulaires (25) réparties sur toute sa longueur en étant espacées axialement.

2. Cheville osseuse (10) selon la revendication 1, **caractérisée en ce que** les nervures annulaires (25) sont réalisées en angle aigu et à la manière de barbes, et présentent un flanc abrupt (26) pointant vers l'extrémité supérieure et un flanc plat (28) pointant vers l'extrémité inférieure.

3. Cheville osseuse (10) selon les revendications 1 et 2, dont l'enveloppe de cheville présente en outre des nervures longitudinales (22) et des ailes longitudinales (30) faisant fonction de sécurité anti-rotation.

4. Cheville osseuse (10) selon les revendications 1 à 3, dont les ailes longitudinales (30) s'étendent chaque fois entre les nervures annulaires (25a), leur arête extérieure s'étendant au niveau de la hauteur radiale maximale des nervures annulaires.

5. Cheville osseuse (10) selon la revendication 4, dont les ailes longitudinales (30) adjacentes dans la direction axiale sont décalées les unes par rapport aux autres dans la direction du pourtour.

6. Cheville osseuse (10) selon la revendication 2, dont les flancs plats (28) pointant vers l'extrémité inférieure et réalisés en forme de cône s'étendent chaque fois jusqu'au flanc abrupt (26) de la nervure annulaire (25a) suivante.

7. Cheville osseuse (10) selon la revendication 1, dont le chanfrein est réalisé en tant qu'ouverture d'introduction en forme de V (32) de la fente longitudinale (16).

8. Cheville osseuse (10) selon les revendications 1 et 3, dont l'enveloppe de cheville présente dans la partie supérieure des nervures longitudinales (22) dont la hauteur décroît de la collerette (18) vers la première nervure transversale.

9. Cheville osseuse (10) selon la revendication 1, dont la dernière nervure annulaire se transforme, dans la partie inférieure, en une extrémité de cheville renflée (34).

10. Cheville osseuse (10) selon les revendications 1 à 9, dont la cheville et/ou la vis sont composées d'un matériau résorbable.
